# EUROPEAN PATENT APPLICATION

(11) **EP 4 806 258 A1**
(43) Date of publication of application: **16.09.2026**
(21) Application number: 24888839.8
(22) Date of filing: 11.11.2024
(51) Int. Cl.: A01G 7/00, A01C 1/06, A01G 2/10

(54) **METHOD FOR CULTIVATING PLANT, PLANT PRODUCED THEREBY, AND COMPOSITION FOR IMPROVING GROWING CONDITION OF PLANT**

(30) Priority: 10.11.2023 JP 2023192519
(71) Applicant: National Institute of Advanced Industrial Science and Technology, Chiyoda-ku Tokyo 100-8921 (JP)
(72) Inventor: KANNO, Manabu, Tsukuba-shi, Ibaraki 305-8560 (JP); CHUNG, Kwimi, Tsukuba-shi, Ibaraki 305-8560 (JP); SAKAMOTO, Shingo, Tsukuba-shi, Ibaraki 305-8560 (JP); TAMAKI, Hideyuki, Tsukuba-shi, Ibaraki 305-8560 (JP); MITSUDA, Nobutaka, Tsukuba-shi, Ibaraki 305-8560 (JP); MORIWAKI, Kousuke, Tsukuba-shi, Ibaraki 305-8560 (JP)
(74) Representative: advotec.
(86) International application number: PCT/JP2024/040039
(87) International publication number: WO 2025/100548

(57) **Abstract**

Provided is a novel plant cultivation method utilizing microorganisms, that can promote plant growth and increase harvest yield and biomass volume. An actinomycete belonging to *Streptomyces thermocarboxydus* is applied to the plant or its propagating material.

## Description

### FIELD

The present invention relates to a plant cultivating method and a plant produced by the method, as well as to a composition for enhancing the growth condition of a plant.

### BACKGROUND

With increasing social demand in recent years for obtaining stable food supplies needed to feed the worldwide population, and reducing greenhouse gases by reinforcing the ability of plants to sequester carbon dioxide (CO₂), it has been a goal to achieve greater harvest yields and plant biomass volume of plants including agricultural crops, feed crops and trees, and this has spurred increasingly active research and development in the field of plant science. However, the use of plants developed using gene recombinant technology and genome editing technology, either as outdoor plants or as food sources, is becoming more difficult to implement in Japan both from a legal and social perspective, due to concerns regarding their effects on the human body and ecological systems. Moreover, in Japan there has been a push to limit the use of chemical fertilizers, with increasing focus on more sustainable agriculture with a reduced environmental load, which has made it even more difficult to carry out highly productive plant cultivation.

For this reason, plant cultivation processes utilizing microorganisms have recently become a subject of focus. A large variety of microorganisms generally co-exist and live symbiotically throughout the surface layers and interiors of the rhizospheres and leaves of outdoor plants, being closely involved in their productivity and growth, and demand for cultivation methods that make use of such microorganisms is increasing. Examples of such useful microorganisms include various strains of actinomycetes belonging to the genus *Streptomyces.*

PTL 1 (Japanese Unexamined Patent Publication No. 2011-188761) describes *Streptomyces actinomycetes* strain MBFA-172 (Accession No. NITE P-896), having an effect of controlling strawberry anthracnose, as well as a biological material for prevention of diseases which comprises the strain.

NPL 1 (Passari et al. PloS one, (2019), 14.7:e0219014) describes treating tomato (*Solanum lycopersicum*) seedlings with the endophytic actinomycete *Streptomyces thermocarboxydus-*separated strain BPSAC147 and cultivating under greenhouse conditions, also teaching that the measured photochemical quantum yield and electron transport rate in Photosystem II (PSII) indicates improvement in the photosynthesis process, and this microorganism is expected, though not yet proven, to serve as a novel biological material for improved productivity via the mechanism of promoting photosynthesis.

NPL 2 (Lasudee et al., Frontiers in Microbiology, (2018), 9:1247) teaches that the arbuscular mycorrhizal fungal strain *Streptomyces thermocarboxydus* S3 isolated from *Funneliformis mosseae* spores, has excellent phosphoric acid solubilizing power, indole-3-acetic acid (IAA) producing ability and siderophore producing ability, that the microorganism significantly increased fresh weight, root length and full length when cultivated by treatment with mung bean (*Vigna radiata*), and that treatment of rice (jasmine rice) with the microorganism and cultivation under dry stress in low-nutritive soil promoted growth in the initial stage up to 45 days of cultivation.

In NPL 3 (Kanno et al., Environ. Microbiol., 2016,18[8]:2495-2506), the present inventors have also reported on the actinomycetes strain *Streptomyces thermocarboxydus* OS2C (Receipt No.: NITE ABP-03739), purely isolated from paddy rice stems which have had their plant surfaces sterilized. Strain OS2C is a plant symbiotic microorganism having a specific enzyme gene that oxidizes dilute hydrogen at the level of atmospheric concentration, and having the ability to oxidize hydrogen with high affinity. In the same publication, the present inventors reported that when rice seeds are treated with this OS2C strain and cultivated in sterile soil, the strain becomes localized in the roots and stems, suggesting that OS2C is a plant endophyte. It was further reported that after sterile soil cultivation for approximately 4 weeks (approximately the 5th week of germination), the lengths and dry weights of the shoots and roots of the rice had increased compared to untreated plants.

### [CITATION LIST]

### [PATENT LITERATURE]

[PTL 1] Japanese Unexamined Patent Publication No. 2011-188761

### [NON PATENT LITERATURE]

[NPL 1] Passari et al.. PloS one, (2019), 14.7:e0219014
[NPL 2] Lasudee et al., Frontiers in Microbiology, (2018), 9:1247
[NPL 3] Kanno et al., Environ. Microbiol., 2016, 18[8]:2495-2506
[NPL 4] Isawa et al., Microbes and Environments, (2009), 1001180152-1001180152
[NPL 5] Gravel et al., Soil Biol. Biochem., (2007), 39.8:1968-1977:
[NPL 6] Saito et al., Nihon Sakumotsu Gakkai Journal, (2000), 69.3:385-390
[NPL 7] Wagner et al., Nat. Commun., (2016), 7:12151

### SUMMARY

### [TECHNICAL PROBLEM]

However, all of the aforementioned previous reports relating to *Streptomyces thermocarboxydus,* as a type of *Streptomyces,* are limited to describing the effects on the growth rate and stress resistance during the vegetative growth stage, when cultivated in a soil cultivation system with microbial treatment of plants having an annual life cycle. No published reports yet exist regarding the effects of actinomycete treatment of harvest yields or biomass yields obtained by cultivation of annual plants up to the harvest stage, or the effect of actinomycete treatment in cultivating systems derived from perennial plants such as trees, or their cuttings.

The present invention has been accomplished in light of this situation, and its object is to provide a novel plant cultivation method utilizing microorganisms, that can promote plant growth and improve harvest yield and biomass. More specifically, it is an object of the invention to provide a novel plant cultivation method that can increase harvest yield and biomass volume when an annual plant is cultivated up to the harvest stage. It is another object of the invention to provide a novel plant cultivation method that can promote plant growth and increase crop harvest yield and biomass volume, in cultivation systems from perennial plants such as trees, or their cuttings.

### [SOLUTION TO PROBLEM]

As a result of much diligent research, the present inventors have found if an actinomycete belonging to *Streptomyces thermocarboxydus* is applied to a plant or its propagating material and the plant is cultivated, it is possible to improve the growth condition of the plant, promoting plant growth and increasing harvest yield and biomass volume. In particular, it was found that it is thereby possible to obtain an effect of increased harvest yield and biomass volume during the harvest stage of annual plants, or an effect of promoting growth, or of suppressing dormancy and preventing growth arrest, in cultivation systems derived from perennial plants such as trees or their cuttings, and the invention has been completed upon this finding.

Specifically, the present invention encompasses the following.

### [Aspect 1]

A method for cultivating a plant, the method comprising applying an actinomycete belonging to *Streptomyces thermocarboxydus* to the plant or its propagating material.

### [Aspect 2]

The method according to aspect [1], wherein the plant is an annual plant, and the actinomycete is applied to the plant from the 6th week after germination.

### [Aspect 3]

The method according to aspect [1], wherein the plant is a perennial plant.

### [Aspect 4]

The method according to any one of aspects [1] to [3], wherein the plant propagating material is part or all of the plant.

### [Aspect 5]

The method according to any one of aspects [1] to [4], wherein the part of the plant is selected from among cells, tissue, organs, seeds, bulbs, cuttings and seedlings.

### [Aspect 6]

The method according to any one of aspects [1] to [5], wherein the actinomycete is strain OS2C (Receipt No.: NITE ABP-03739) or a variant thereof.

### [Aspect 7]

The method according to any one of aspects [1] to [6], wherein application of the actinomycete is accomplished by direct application of the actinomycete to the plant or its propagating material, or by indirect application of the actinomycete to the surrounding environment of the plant or its propagating material.

### [Aspect 8]

The method according to any one of aspects [1] to [7], wherein application of the actinomycete improves the growth condition of the plant.

### [Aspect 9]

The method according to aspect [8], wherein the improvement in the growth condition of the plant is increased crop harvest yield, increased plant biomass volume, promoted root formation, promoted development of roots, stems or leaves, suppressed dormancy of the plant, suppressed growth arrest of the plant, or any combination of the foregoing.

### [Aspect 10]

A plant or its propagating material, created by a method according to any one of aspects [1] to [9].

### [Aspect 11]

A composition comprising an actinomycete belonging to *Streptomyces thermocarboxydus,* which is used for improving the growth condition of a plant.

### [Aspect 12]

The composition according to aspect [11], wherein the actinomycete is strain OS2C (Receipt No.: NITE ABP-03739) or a variant thereof.

### [Aspect 13]

The composition according to aspect [11] or [12], wherein the improvement in the growth condition of the plant is increased crop harvest yield, increased plant biomass volume, promoted root formation, promoted development of roots, stems or leaves, suppressed dormancy of the plant, suppressed growth arrest of the plant, or any combination of the foregoing.

### [Aspect 14]

The composition according to any one of aspects [11] to [13] above, wherein the plant is an annual plant, and the composition is applied to the plant from the 6th week after germination.

### [Aspect 15]

The composition according to any one of aspects [11] to [13], wherein the plant is a perennial plant.

### [ADVANTAGEOUS EFFECTS OF INVENTION]

The plant cultivation method using an actinomycete according to the invention can promote plant growth and increase harvest yield and biomass volume, by application to and cultivation of a plant or its propagating material. In particular, the plant cultivation method according to one aspect of the invention can provide an effect of increased harvest yield and biomass volume during the harvest stage for an annual plant. In addition, the plant cultivation method according to one aspect of the invention can provide effects such as promoting growth in a cultivation system derived from a perennial plant such as a tree or its cuttings, or suppressing dormancy or preventing growth arrest, as well as increasing crop harvest yield and biomass volume.

### BRIEF DESCRIPTION OF DRAWINGS

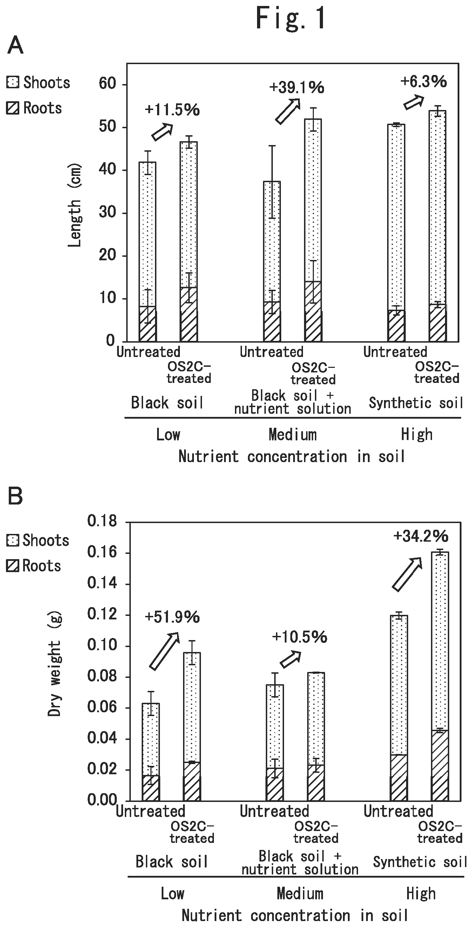

Fig. 1A and Fig. 1B are graphs showing the average values for length and dry weight of rice (respective measured values for the shoots and roots of each plant) 22 days after treatment with strain OS2C (29 days after germination) with cultivation in three soils having different nutrient concentrations, in comparison to untreated rice, in the pot test of Example 2.

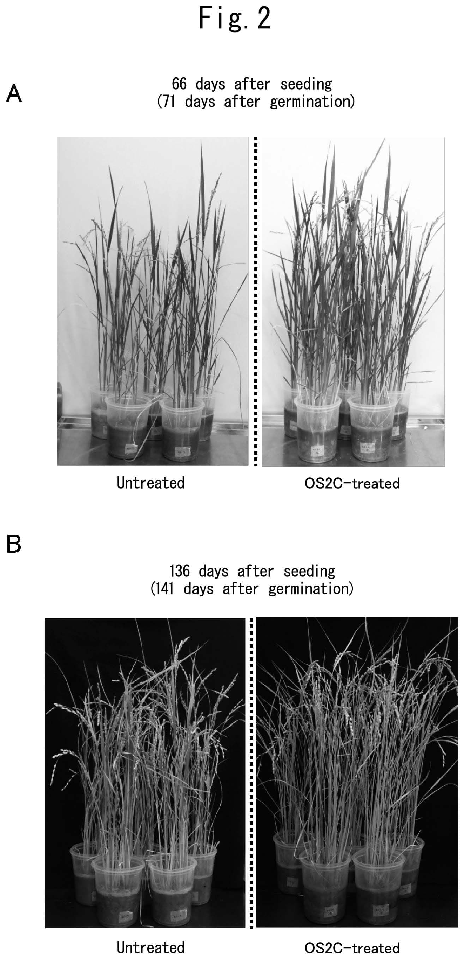

Fig. 2A and Fig. 2B are photographs showing full images of rice at 66 days after treatment with strain OS2C (71 days after germination, heading stage) and 136 days after treatment (141 days after germination, harvest stage), in comparison to untreated rice during the same period.

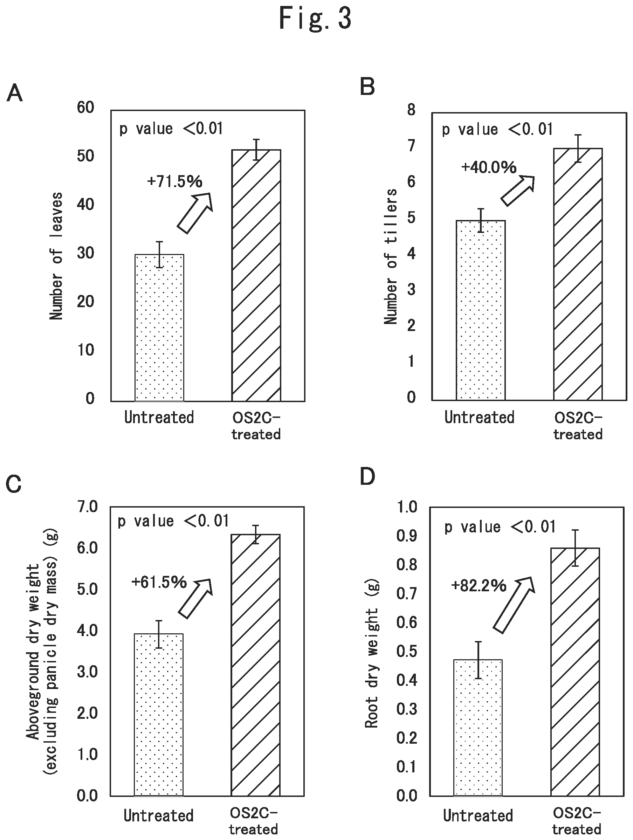

Fig. 3 shows four graphs: Fig. 3A showing the average value for number of rice leaves, Fig. 3B showing the average value for number of tillers, Fig. 3C showing the average value for shoot dry weight excluding the panicle dry weight (about 0.1 to 0.2 g each), and Fig. 3D showing the average value for root dry weight, at 51 days after treatment with strain OS2C (56 days after germination, immediately after the heading stage), in comparison to untreated rice.

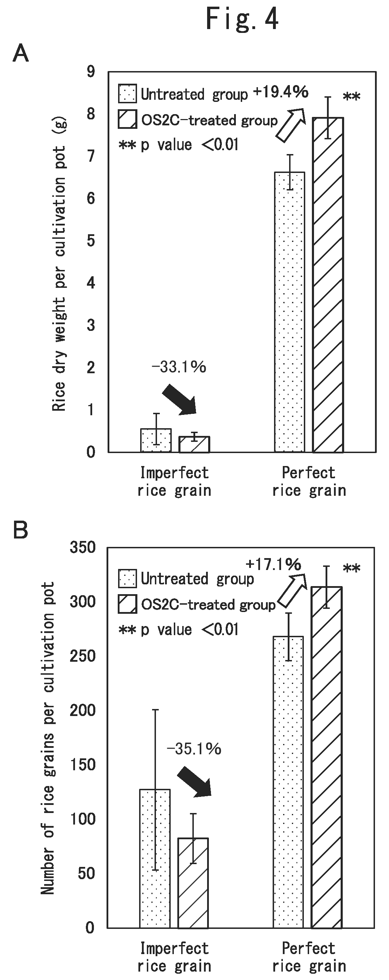

Fig. 4A and Fig. 4B are graphs showing the average dry weight of rice per cultivation pot harvested in the OS2C-treated group, and the average number of rice grains per cultivation pot, divided into perfect rice grains and imperfect rice grains, in comparison to untreated rice.

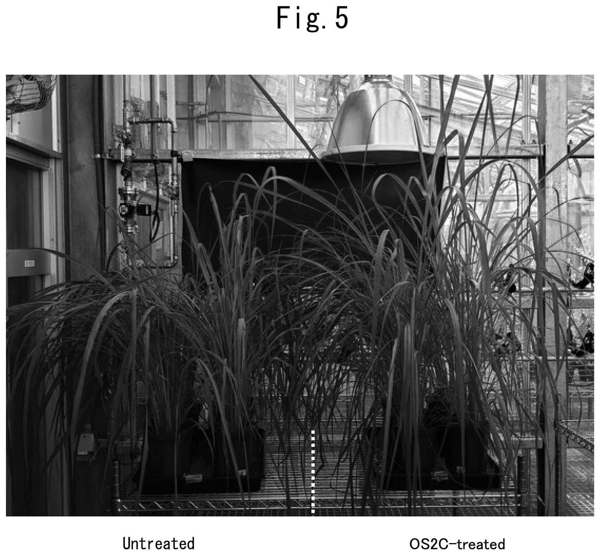

Fig. 5 is a photograph showing a full image of pot-cultivated erianthus after 123 days of treatment with strain OS2C (139 days after seeding), in comparison to untreated erianthus.

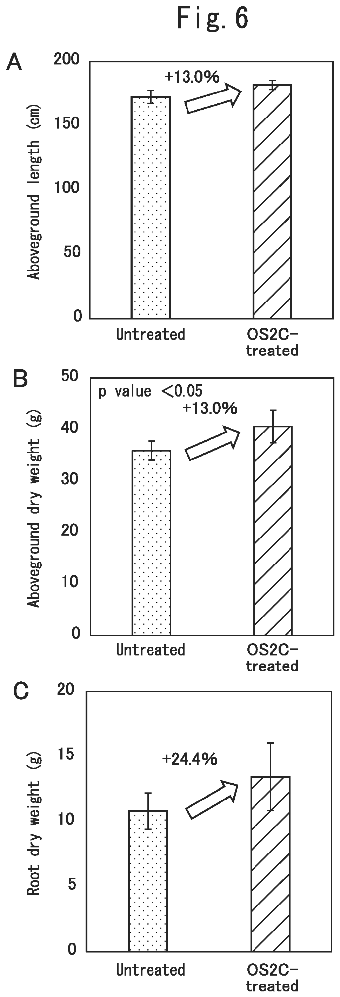

Fig. 6A is a graph showing the average value for shoot length per erianthus plant, at 123 days after treatment with strain OS2C (139 days after seeding), in comparison to an untreated plant. Fig. 6B is a graph showing the average value for shoot dry weight of OS2C-treated erianthus for the same period, in comparison to an untreated plant. Fig. 6C is a graph showing the average value for root dry weight of OS2C-treated erianthus for the same period, in comparison to an untreated plant.

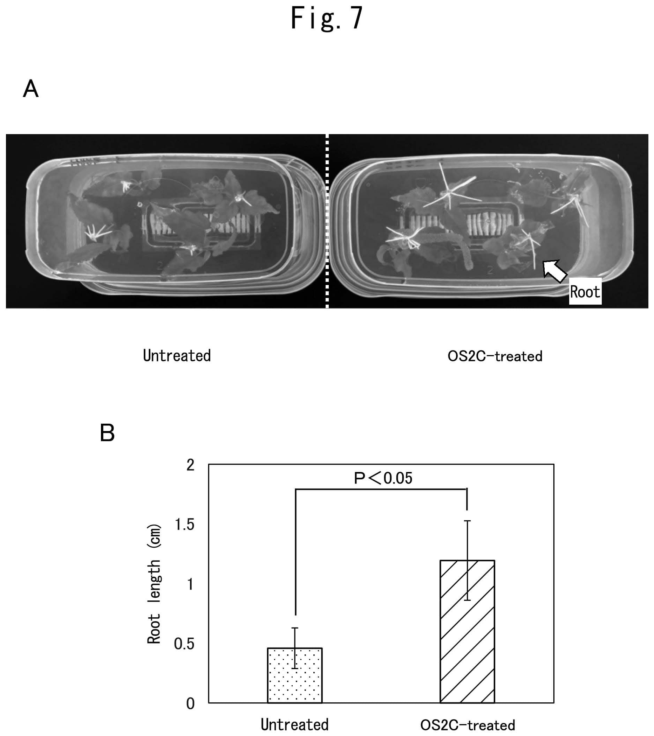

Fig. 7A is a photograph showing the container bottom of a plant after 10 days of treatment of strain OS2C during subculturing of fresh medium using a portion of the organ including the growth point from sterile-cultured poplar, compared to a untreated plant. Fig. 7B is a graph showing a comparison of the average value for root length of each plant in Fig. 7A.

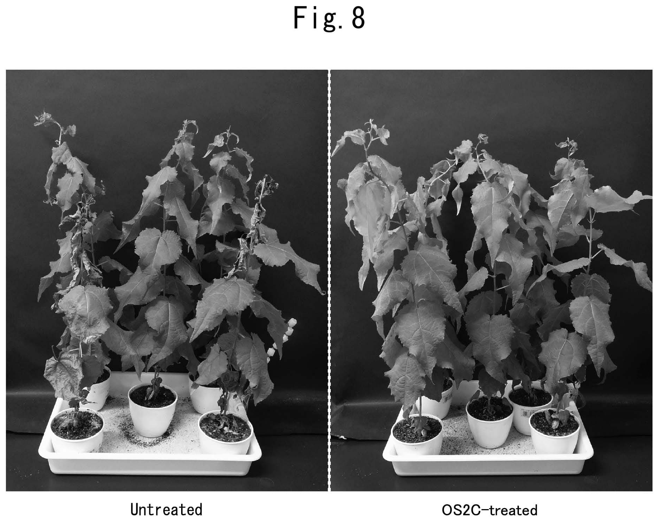

Fig. 8 is a pair of photographs showing full images at 88 days after initial treatment for OS2C-treated poplar (46 days after pot transplant), in comparison to the untreated poplar.

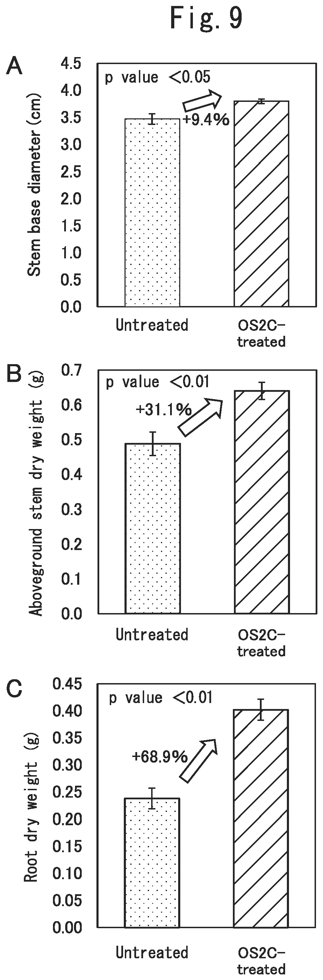

Fig. 9A is a graph showing the average value for stem base diameter of OS2C-treated poplar, 88 days after initial treatment with strain OS2C (46 days after pot transplant), in comparison to untreated poplar. Fig. 9B is a graph showing the average value for shoot stem dry weight of OS2C-treated poplar for the same period, in comparison to untreated poplar. Fig. 9C is a graph showing the average value for root dry weight of OS2C-treated poplar for the same period, in comparison to untreated poplar.

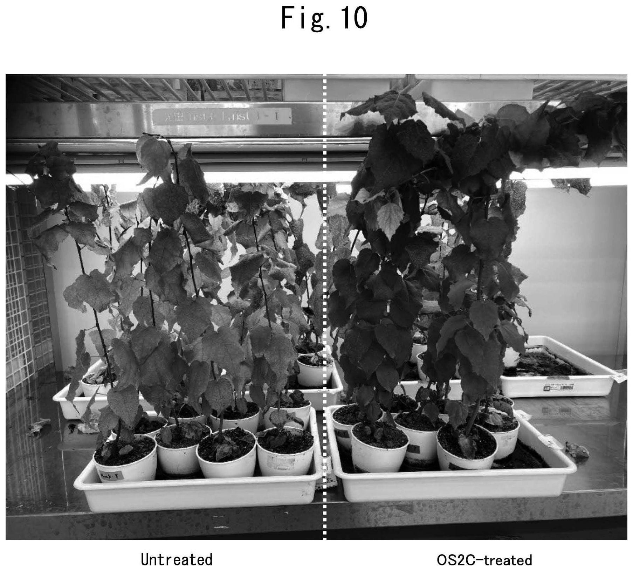

Fig. 10 is a pair of photographs showing full images at about 4 months after pot transplant of OS2C-treated poplar, in comparison to untreated poplar.

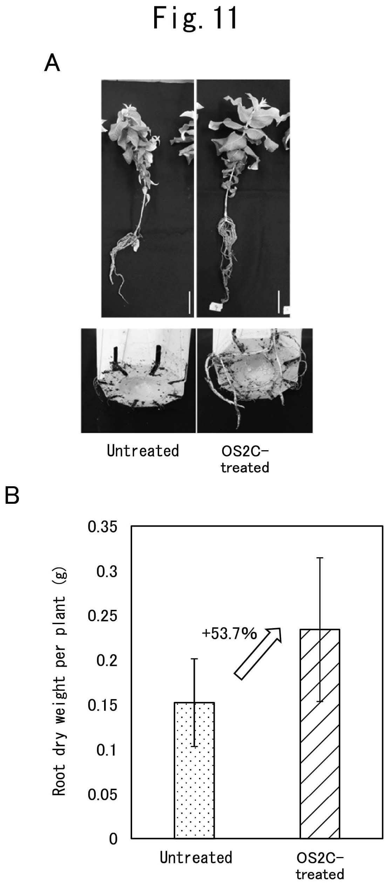

Fig. 11A shows, at top, full images of a eucalyptus plant 142 days after initial treatment with strain OS2C (152 days after seeding), and at bottom, photographs showing the state of the roots in the pot, in comparison to an untreated plant. Fig. 11B is a graph showing the average value for root dry weight per plant of OS2C-treated eucalyptus for the same period, in comparison to an untreated plant.

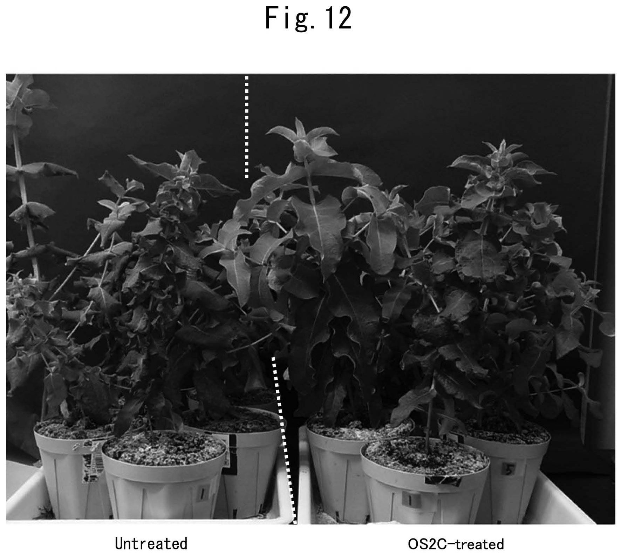

Fig. 12 is a photograph showing a full image of pot-cultivated eucalyptus at 91 days after initial treatment with strain OS2C (105 days after seeding), in comparison to an untreated plant.

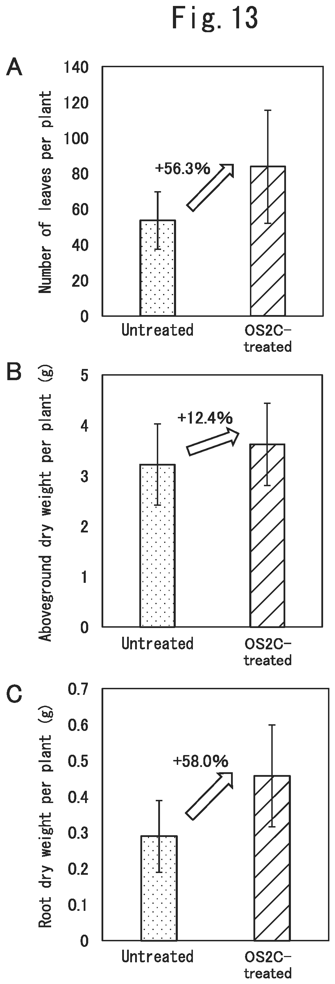

Fig. 13A is a graph showing the average value for number of leaves per plant, at 91 days after initial treatment with strain OS2C (105 days after seeding), in comparison to an untreated plant. Fig. 13B is a graph showing the average value for shoot dry weight per plant for the same period, in comparison to an untreated plant. Fig. 13C is a graph showing the average value for root dry weight per plant for the same period, in comparison to an untreated plant.

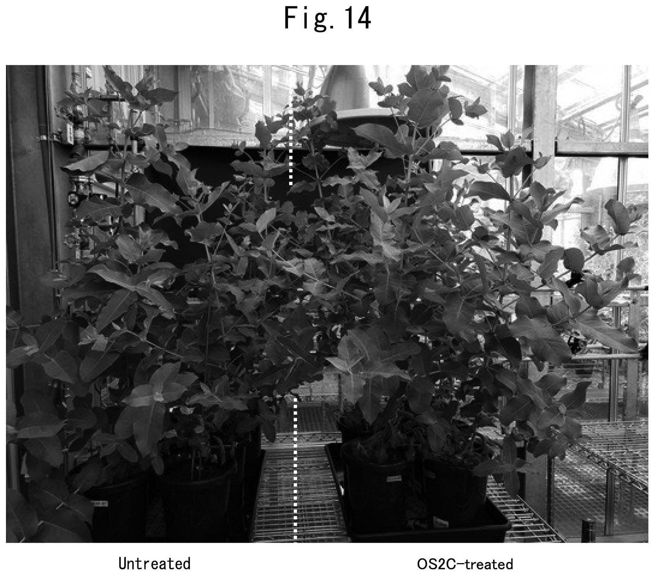

Fig. 14 is a photograph showing a full image of pot-cultivated eucalyptus at 159 days after initial treatment with strain OS2C (180 days after seeding), in comparison to an untreated plant.

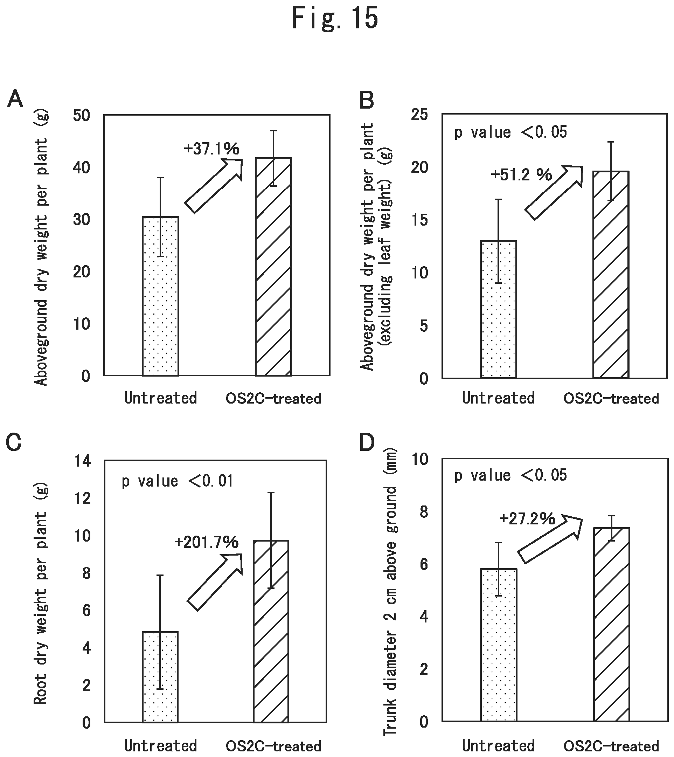

Fig. 15A is a graph showing the average value for aboveground dry weight per eucalyptus plant, 159 days after initial treatment with strain OS2C (180 days after seeding), in comparison to an untreated plant. Fig. 15B is a graph showing the average value of the dry weights of aboveground parts per eucalyptus plant, for stems without leaves, during the same period in comparison to an untreated plant. Fig. 15C is a graph showing the average value for root dry weight per eucalyptus plant for the same period, in comparison to an untreated plant. Fig. 15D is a graph showing average value for trunk diameter at a location 2 cm from the ground surface, per eucalyptus plant during the same period, in comparison to an untreated plant.

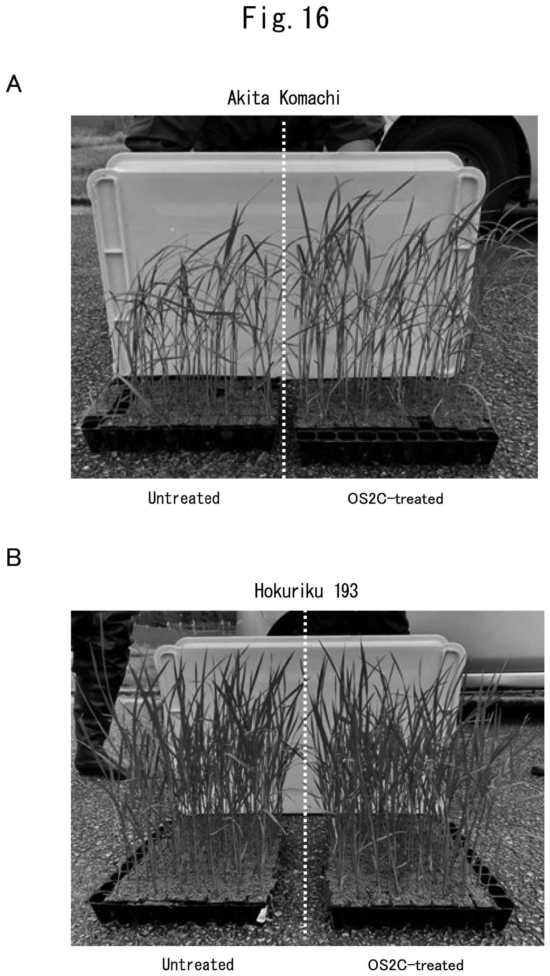

Fig. 16A and Fig. 16B are photographs showing full images of rice (Akita Komachi and Hokuriku 193) at 19 days after treatment with strain OS2C (26 days after germination), in comparison to untreated rice during the same period.

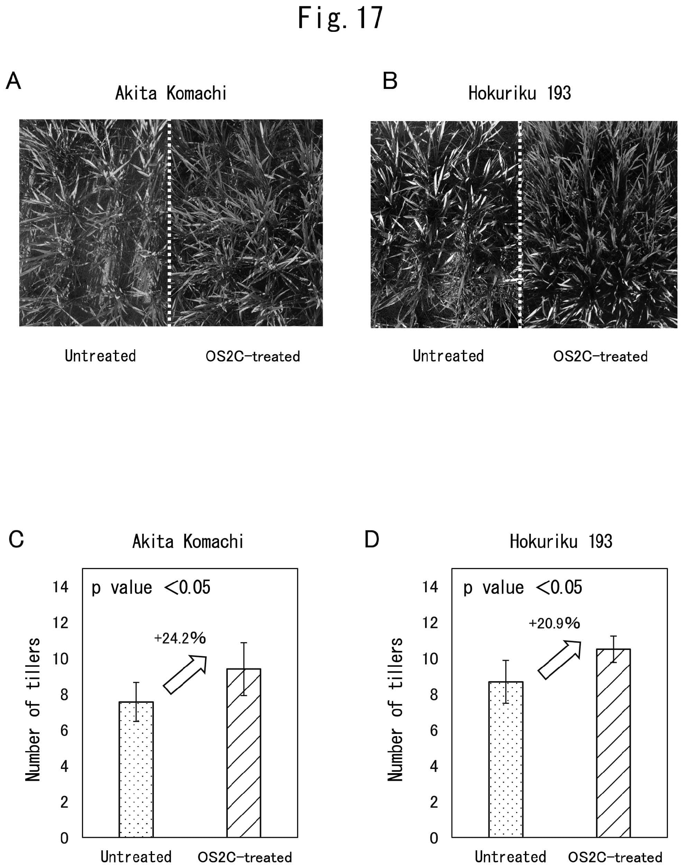

Fig. 17A and Fig. 17B are photographs with full images of rice (Akita Komachi and Hokuriku 193) at 78 days after treatment with strain OS2C (59 days after paddy transplant), from above the paddy, showing growth in comparison to untreated rice during the same period. Fig. 17C and Fig. 17D are graphs showing the average number of tillers of OS2C-treated rice plants (Akita Komachi and Hokuriku 193) 78 days after treatment with strain OS2C (59 days after paddy transplant), in comparison to untreated rice.

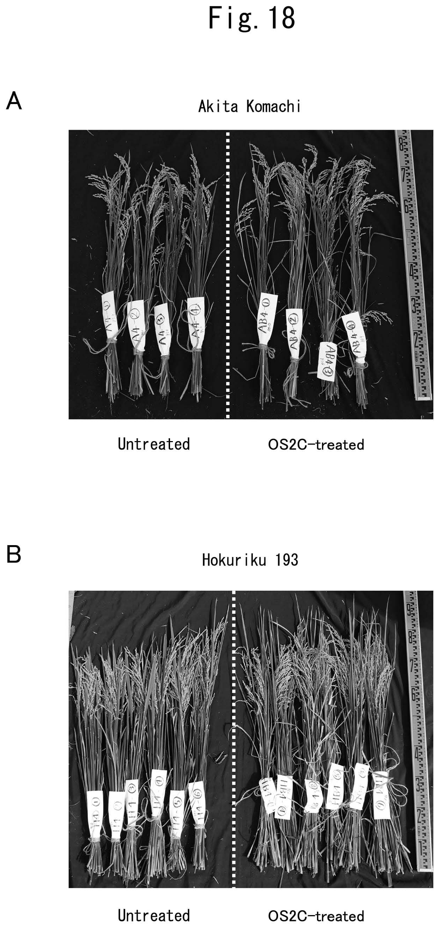

Fig. 18A and Fig. 18B are photographs showing rice (Akita Komachi and Hokuriku 193) harvested 155 days after treatment with strain OS2C (136 days after paddy transplant), in comparison to untreated rice during the same period.

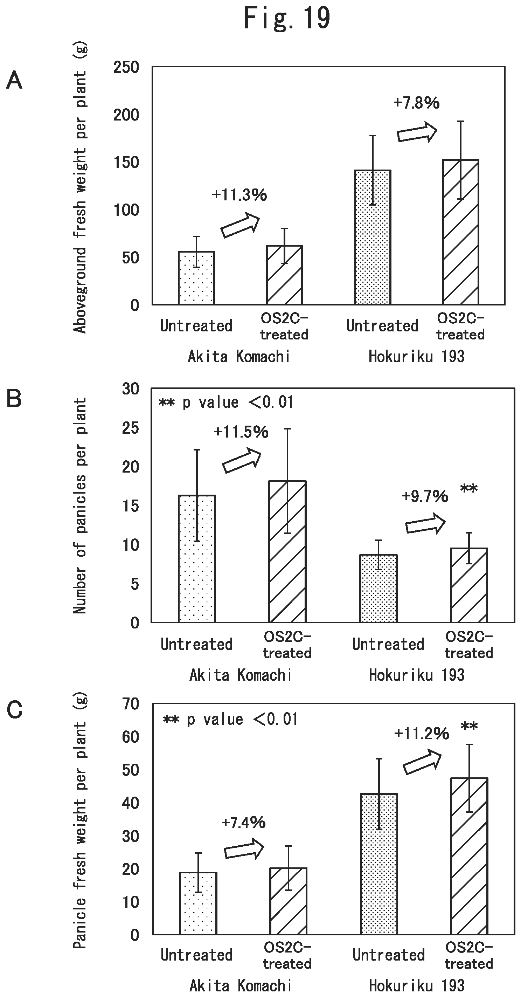

Fig. 19 is a set of graphs, Fig. 19A showing the average values for fresh shoot mass per plant, Fig. 19B showing the average number of panicles per plant, and Fig. 19C showing the average values for fresh panicle mass per plant, for rice (Akita Komachi and Hokuriku 193) harvested 155 days after treatment with strain OS2C (136 days after paddy transplant), in comparison to untreated plants.

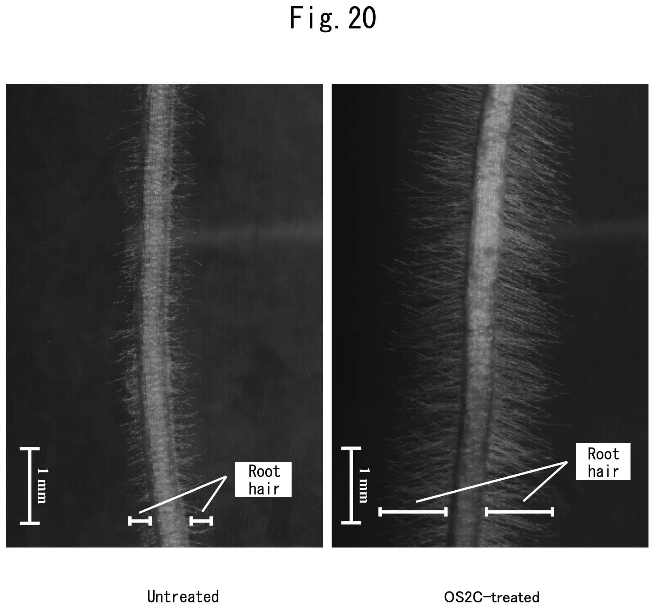

Fig. 20 is a pair of photographs showing the root hairs of the first seed root at a distance of 4 cm from the rice seeds 3 days after treatment with strain OS2C (6 days after seeding), in comparison to untreated rice.

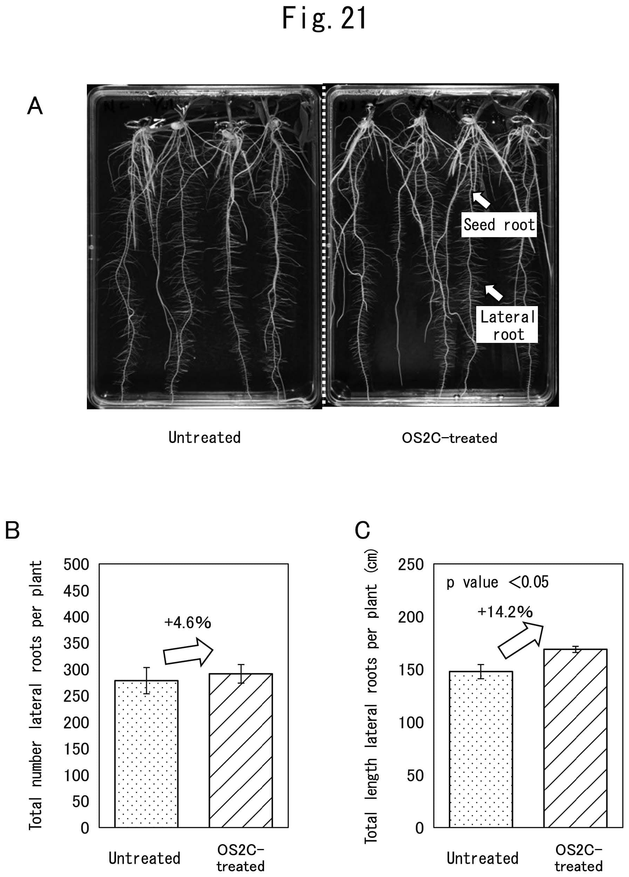

Fig. 21A is a pair of photographs showing full images of root development of rice 7 days after treatment with strain OS2C (10 days after seeding), in comparison to untreated rice. Fig. 21B and Fig. 21C are graphs showing the total numbers of lateral roots and the lengths of lateral roots per rice plant, 7 days after treatment with strain OS2C (10 days after seeding), in comparison to untreated rice.

### DESCRIPTION OF EMBODIMENTS

The invention will now be described in greater detail by concrete embodiments. However, the invention is not restricted to the described embodiments and may be carried out in any form within a range that is not outside of the gist of the invention.

All of the references including patent literature (such as patent applications and patent publications) and non-patent literature cited for the purpose of the invention are invoked in their entirety and are incorporated herein for all purposes.

One aspect of the invention relates to a method for cultivating a plant, the method comprising applying an actinomycete belonging to *Streptomyces thermocarboxydus* to the plant or its propagating material (this will hereunder be referred to as "method of the invention", as appropriate).

As mentioned above, several previous reports, including one by the present inventors, have described cultivation of plants treated with different actinomycete strains of *Streptomyces* (see PTL 1 and NPLs 1 to 3). However, these previous reports have all been limited to describing the effects on growth rate and stress resistance in the vegetative growth stage, when cultivating annual plants in a soil cultivation system with treatment using microbial strains. No published reports exist in the prior art regarding the effects of actinomycete treatment of harvest yields or biomass yields obtained by cultivation of annual plants up to the harvest period, or the effect of actinomycete treatment in cultivating systems derived from perennial plants such as trees, or their cuttings.

The present inventors have found that when actinomycetes belonging to *Streptomyces thermocarboxydus* (OS2C described below) are used to treat an annual plant (rice) and cultivated by indoor soil cultivation up to the harvest stage, the yield and biomass volume are notably increased (see Example 2 and Figs. 1 to 4). It has been suggested that increased growth of annual plants during the vegetative growth stage and increased harvest yield and biomass volume during the harvest stage do not necessarily occur by the same action mechanism (see NPL 4: Isawa et al., Microbes and Environments, (2009), 1001180152-1001180152; NPL 5: Gravel et al., Soil Biol. Biochem., (2007), 39.8:1968-1977: NPL 6: Saito et al., Nihon Sakumotsu Gakkai Journal, (2000), 69.3:385-390.). Therefore, the increased harvest yield and biomass volume in the harvest stage obtained by treatment and cultivation of annual plants with actinomycetes belonging to *Streptomyces thermocarboxydus* was a surprising finding that could not at all be easily imagined from previous reports.

The present inventors later found that when actinomycetes belonging to *Streptomyces thermocarboxydus* (the OS2C described below) are used to treat an annual herbaceous resource crop (erianthus) which is then cultivated by soil cultivation, the biomass volume is notably increased (see Example 3 and Figs. 5 and 6). It was also surprising to discover that actinomycetes belonging to *Streptomyces thermocarboxydus* that have been isolated from agricultural crops also contribute to increased biomass volume of resource crops.

The present inventors also carried out treatment of seeds of an annual plant (rice) with actinomycetes belonging to *Streptomyces thermocarboxydus* (the OS2C described below), and carried out soil cultivation. Stems including a growth point and several leaves were cut out from a perennial plant (poplar) during aseptic culturing and subcultured in new medium, treated with the same OS2C strain and cultured, and then transplanted into pots and further cultivated. The same strain OS2C was used to treat seeds of a perennial plant (eucalyptus) which were cultured in agar medium, and then potted before continued cultivation. As a result, it was found that all of the effects of promoting growth, suppressing dormancy, preventing growth arrest and increasing biomass volume can be obtained (see Example 4 and Figs. 7 to 10). It is known that the community structure of symbiotic microorganisms differs depending on the number of years of growth of a plant (NPL 7: Wagner et al., Nat. Commun., (2016), 7:12151), and it is conjectured that the action mechanism for growth promotion differs between annual plants and perennial plants. Moreover, since growth of plants in a soil cultivation system, and differentiation and generation in cultivation systems other than soil, such as tissue culture and cuttings, generally have completely different physiological mechanisms in plants, they are believed to occur due to different growth-promoting action mechanisms. It was therefore surprising knowledge, which could not be easily obtained from previous reports, that in subculturing using organ parts from perennial plants (such as poplar) during aseptic culturing, it is possible to obtain effects such as promoting subsequent growth, suppressing dormancy, preventing growth arrest and increasing biomass volume by cultivation after treatment with actinomycetes belonging to *Streptomyces thermocarboxydus.*

The present inventors later found that when actinomycetes belonging to *Streptomyces thermocarboxydus* (the OS2C described below) were used to treat a perennial tree resource crop (eucalyptus) which is then cultivated by soil cultivation, the biomass volume is notably increased (see Example 5 and Figs. 11 to 15). Based on the background described above, the finding that actinomycetes belonging to *Streptomyces thermocarboxydus* similarly contribute to increased biomass volume of tree resource crops is surprising knowledge which could not be easily imagined from previous reports.

Examples of actinomycetes belonging to *Streptomyces thermocarboxydus* for the method of the invention include strain OS2C, and its variants. OS2C is a microorganism that has been purely isolated by the present inventors from paddy rice stems after sterilization of the plant surfaces, and it is a plant symbiotic microorganism having a specific enzyme gene that oxidizes dilute hydrogen at the level of atmospheric concentration, and having the ability to oxidize hydrogen with high affinity. When OS2C was used to treat individual plants and cultured, localization was observed in the roots and stems (NPL 3), suggesting that OS2C is an endophyte. Based on sequence homology analysis of the 16S rRNA gene, as a marker gene for bacterial phylogenetic systematics, it has been shown that the sequence of the 16S rRNA gene of OS2C (Accession No. AB894408) has 99.9% identity with strain AT37 of *Streptomyces thermocarboxydus* (Accession No. NR026072), which is the most closely related strain.

The type of plant used for the method of the invention is not restricted. As classified by breeding method, for example, the plant may be a spermatophyte, or a plant such as moss or fern which does not produce seeds. In the case of a spermatophyte, it may be an angiosperm or a gymnosperm. In the case of an angiosperm, it may be a monocotyledon or a dicotyledon. As classified by life cycle, the plant may be an annual plant or a perennial plant. There are also no restrictions on the use of the plant, but it is preferably a crop plant such as an agricultural crop or resource crop, or an economically important plant such as a houseplant or garden plant.

For the method of the invention, there is no restriction on the form of the plant which is to be treated with the actinomycete. For example, application may be to the plant after germination, or to a propagating material of the plant before germination. Plant propagating materials that may be used include all or parts of plants such as cells, tissues, organs, seeds, bulbs, cuttings and seedlings. Plant cells and tissue may be in the differentiated state as obtained from the plant, or in the form of stem cells or a callus state after dedifferentiation. Various methods are known for dedifferentiation of plant cells and tissues.

The plant cultivation system used for the method of the invention is also not particularly restricted. Examples include soil culture systems using various types of soils, and hydroponic systems using nutrient solutions instead of soil. In the case of a soil culture system, the soil may be natural soil or artificial soil. A hydroponic system may be a solid cultivation system using any of various solid media, or a liquid cultivation system using water or any of various liquid media (a hydroponic cultivation system). Any of these systems may be supplemented with a fertilizer such as natural fertilizer or artificial fertilizer, or various nutrients. Cultivation in the seed germination stage or the seedling vegetative growth stage may be in a solid cultivation system or hydroponic system, with potting and transfer to a soil culture system after a certain degree of growth of the plant, and a combination of multiple cultivation systems may also be employed.

There are no particular restrictions on the stage or the number of times the actinomycetes are applied to the plant or its propagating material in the method of the invention. Application to a germinated plant, for example, may be at any desired stage (for example, the germination stage, vegetative growth stage or reproductive growth stage), or at multiple different stages (for example, two or more from among the germination stage, vegetative growth stage and reproductive growth stage). Application may also be to a propagating material at any stage before germination, or to a cultivation system such as the soil used for cultivation of a plant. For each application period, the actinomycete may be applied just once, or two or more times.

For an annual plant, however, the actinomycete is preferably applied at least during the period after the vegetative growth stage, and specifically it is applied onto the plant from the 6th week after germination. As mentioned above, it has been suggested that increased growth of annual plants during the vegetative growth stage and increased harvest yield and biomass volume during the harvest stage do not necessarily occur by the same action mechanism (see NPL 4: Isawa et al., Microbes and Environments, (2009), 1001180152-1001180152; NPL 5: Gravel et al., Soil Biol. Biochem., (2007), 39.8:1968-1977: NPL 6: Saito et al., Nihon Sakumotsu Gakkai Journal, (2000), 69.3:385-390.). Applying the actinomycete to an annual plant after the vegetative growth stage can provide a notable effect of increased harvest yield and biomass volume during the harvest stage, which has been completely unknown in the prior art. When an actinomycete is applied to an annual plant, however, it may be applied not only at the stage after the vegetative growth stage (i.e. from the 6th week after germination) but also at a stage before the vegetative growth stage (i.e. up to the 5th week after germination).

There are no particular restrictions on the method of applying an actinomycete to a plant or its propagating material in the method of the invention. Examples include direct application, such as treating the plant or its propagating material by dispersing or coating a culture solution of the actinomycete, or immersion of the plant or its propagating material into a culture solution of the actinomycete, and indirect application such as mixing and absorbing a culture solution of the actinomycete into the surrounding environment of the plant or its propagating material (such as soil, culture medium or water).

For the method of the invention, the amount of actinomycete applied with respect to the plant or its propagating material is not particularly restricted, and it may be appropriately selected depending on the conditions, including the type of plant, and the stage and method of application. As an example, first a culture solution is prepared with the actinomycete prepared to a cell concentration of about 10⁶ to 10¹⁰ CFU/mL. For direct application, the culture solution may be applied in an amount of about 0.1 to 10 mL per plant. For indirect application, the culture solution may be mixed to a concentration of 1 to 100 mL/kg (for soil or solid medium) or 0.01 to 50 mL/L (for liquid medium or water) with respect to the soil, culture medium or water. The actinomycete is prepared in the form of spores or mycelia.

According to the method of the invention, application of an actinomycete to a plant or its propagating material can improve the state of its growth (especially it can improve the state of growth in a manner that does not depend on the cultivation environment, such as the soil). Another aspect of the invention, therefore, provides a composition for improving the state of growth of a plant that comprises the aforementioned actinomycete as an active component.

Improvement in the state of growth may include any of the following effects, without any limitations.
· Increased harvest yield of an agricultural crop (when the plant is an agricultural crop).
· Increase in the biomass volume of a resource crop (when the plant is a resource crop).
· Promoting root formation.
· Promoting development of roots, stems and leaves.
· Suppressing plant dormancy.
· Preventing plant growth arrest.

According to the method of the invention, plants are produced with symbiotic growth of actinomycetes, and exhibit one or more of the aforementioned effects. Plants created by the method of the invention, or their propagating materials, are also encompassed by the present invention.

### EXAMPLES

The present invention will now be explained in further detail by Examples, with the understanding that these are merely provided for convenience of explanation and are not intended to limit the invention in any sense.

### [Example 1: Culturing of actinomycetes strain OS2C and preparation of cell solution]

Actinomycetes strain OS2C (Receipt No.: NITE ABP-03739) belonging to *Streptomyces thermocarboxydus* was aerobically plate cultured in R2A agar medium at 30°C. The microorganism formed a gray spore mat on the agar medium after about 10 days of culturing. A suitable amount of sterilized water was poured onto the plate, the spore mat was scraped off, the collected cell solution was centrifuged, and the supernatant liquid was removed. Sterilized water in an amount equivalent to the previous sterilized water was added, and washing was repeated twice to prepare a spore suspension of the microorganism. The viable cell count in the spore suspension was calculated by the colony counting method, and spore suspensions were prepared to cell concentrations of 10⁹ CFU/mL and 10⁸ CFU/mL for use in treating the rice in Examples 2, 6 and 7 below and for treatment of erianthus, poplar or eucalyptus in Examples 3 to 5. The bacterial solution was prepared for use in plant treatment on the specified day, being stored on ice until use.

### [Example 2: Treatment of rice with actinomycetes strain OS2C]

The rice seeds used (variety: Nipponbare) were sterile seeds obtained by sterilization for 1 minute with 70% ethanol and 30 minutes with 1% sodium hypochlorite, followed by washing treatment, after dehusking of paddy seeds which had been screened by saltwater separation. At 5 days after seeding in 0.5% agar medium (no components other than agar), the rice immediately after germination was transplanted into pots containing 200 g of each of three different soils previously wet-sterilized with an autoclave, using two plants per pot. Three different soils were prepared: black soil, black soil + culture solution (nutrient solution-containing black soil, obtained by adding 20 µL equivalent of liquid fertilizer (trade name: HYPONeX feed solution, HYPONeX) to 200 g of black soil), and synthetic soil (synthetic granular soil, trade name: BONSOL No. 2, Sumitomo Chemical Co., Ltd.), in order of lower to higher nutrient concentration in the soil.

During the same day, the OS2C spore suspension at a cell concentration of 10⁹ CFU/mL prepared in Example 1 was added at 1 mL per rice plant, close to the base of the transplant location. Cultivation of rice for this Example was carried out in a growth chamber (28°C; light cycle: 16 hours light period/8 hours dark period; approximately 22,000 lux), with water supply as appropriate using sterilized water.

As shown in Fig. 1A and Fig. 1B, the plant heights (lengths) and dry weights of the rice (the measured values for the shoot and root sections of each plant) at 22 days after treatment with strain OS2C (29 days after germination) in the pot test of Example 2 were compared with untreated rice, for each of the three soils with different nutrients (n = 2). The OS2C-treated rice had increased average values for body length (shoot + root section) in the low, medium and high nutrient soils, respectively: <41.8 cm to 46.6 cm> (+11.5%), <37.3 cm to 51.9 cm> (+39.1%), <50.7 cm to 53.9 cm> (+6.3%). The average values for the dry weights were also increased, respectively: <0.063 g to 0.096 g> (51.9%), <0.075 g to 0.083 g> (+10.5%), <0.120 g to 0.161 g> (+34.2%). These results demonstrated that even in soil with different nutrient amounts, initial biomass production in rice was stably promoted by symbiotic growth of strain OS2C.

The top cover was removed from the rice cultivated in synthetic soil from the 4th week after microbial treatment, and cultivation was continued up to the harvest stage in a growth chamber (30°C, 70% humidity, light cycle: 11 hours light period/13 hours dark period; 470 ppm CO₂ concentration). After 31 days and 51 days of treatment, the sterilized synthetic granular soil was further treated with microbes by adding to the pot the soil mixed with OS2C spores (approximately 2.0 × 10⁹ CFU per pot) that had been cultured in Example 1.

Rice at 66 days after treatment with strain OS2C (71 days after germination: heading stage) and 136 days after treatment (141 days after germination: harvest stage) were compared with untreated rice during the same period, as shown in Fig. 2A and Fig. 2B (collective photos of 5 pots per test group, with 2 plants per pot, for a total of 10). These results demonstrated that OS2C treatment significantly promoted rice growth at both the heading stage and harvest stage of the rice.

Each of the parameters for plant biomass at 51 days after OS2C treatment (56 days after germination, immediately after initial heading) (the number of leaves, average number of tillers and dry weight (DW) of the shoots excluding the panicle weight (about 0.1 to 0.2 g each), and root section dry weight (DW)), were compared with the untreated rice, as shown in Fig. 3. The total values for each of the two plants per pot were averaged for each test group of 8 pots (N = 8). The results demonstrated that the plant biomass volumes of the shoots and roots significantly increased by OS2C treatment.

The dry weights and numbers of granules of the rice harvested at 136 days after OS2C treatment (141 days after germination, harvest stage), divided by imperfect rice grains and perfect rice grains, were compared between the OS2C-treated plants and untreated plants, as shown in Fig. 4A and Fig. 4B. The total values for each of the two plants per pot were averaged for each test group of 5 pots (N = 5). The results demonstrated that OS2C treatment significantly reduced the dry weight and number of imperfect rice grains, while significantly increasing the dry weight and number of perfect rice grains.

In the OS2C-treated group, the stage from heading to grain maturity was approximately 2 weeks earlier than in the untreated group. These results suggested that some effect in the OS2C-treated plants may have promoted substance diffusion in the rice (distribution of nutrients throughout the harvested products).

### [Example 3: Treatment of erianthus with actinomycetes strain OS2C]

Erianthus seeds (*Erianthus arundinaceus*) were sterilized and washed for 5 minutes using 1/20-diluted Kitchen Haiter, and the sterile seeds were used. After seeding in 1% agar medium containing 1% sucrose and 1/4 MS medium and dark culturing for 2 days at 22°C with the plate upright, it was light cultured for 8 days under conditions with a light cycle of 16 hours light period/8 hours dark period, after which it was transplanted into soil that had been pre-sterilized with an autoclave.

During the same day, an OS2C spore suspension at a cell concentration of 10⁸ CFU/mL was added at 1 mL per erianthus plant, close to the base of the transplant location. After one month, additional treatment was carried out with the same OS2C strain and followed by transplanting into a large-size pot, after which OS2C spore suspension at the same cell concentration was further added at 1 mL per erianthus plant.

As shown in Fig. 5 and Figs. 6A to C, an erianthus cultivation pot at 123 days after initial OS2C treatment (139 days after seeding) was prepared, and the length and dry weights of the aboveground and belowground parts were compared with the untreated plants. The values were increased by approximately 13%, 24% and 13%, respectively. These results suggested that OS2C treatment can contribute to promoting growth of both the aboveground and belowground parts of the herbaceous plant erianthus.

### [Example 4: Treatment of poplar with actinomycetes strain OS2C]

The hybridized poplar (*Populus tremula x tremuloides*) plants used had been aseptically cultured under conditions with a light cycle of 16 hours light period/8 hours dark period, at 22°C, in 1/2 MS solid medium containing 0.3% gellan gum. After using a scalpel to cut the internode at a location about 3 cm from the shoot apex of the plant at about one month after subculturing, a portion of about 1 cm from the cut surface was immersed in the OS2C suspension (10⁷ cells/mL) prepared in Example 1 and allowed to stand for 5 minutes for bacterial treatment. Next, the stem of the treated cultured part was inserted into 1/2 MS solid medium containing 2% sucrose with the plant body being self-supported, culturing was continued under conditions with a light cycle of 16 hours light period/8 hours dark period, at 22°C, while periodically observing the effects of bacterial treatment on root formation and early growth. As a control (untreated group), sterilized water was used instead of the cell suspension, and after immersing the cut cultured part in sterilized water for 5 minutes, it was inserted into 1/2 MS solid medium containing 2% sucrose in the same manner, and observed.

The poplar at 10 days after OS2C treatment was compared with untreated poplar, as shown in Fig. 7A. The root length of the poplar at 10 days after OS2C treatment was compared with the root length of the untreated poplar, as shown in Fig. 7B. The results demonstrated that the root lengths were significantly longer in the OS2C-treated group compared to the untreated plants, with accelerated elongation growth as well.

The poplar was also transplanted from agar medium to a soil pot, and cultivation was continued under artificial lighting. A poplar cultivation pot at 88 days after initial OS2C treatment (46 days after potting) was prepared and the stem base thickness and dry weight and the root dry weight per plant were compared with untreated plants, as shown in Fig. 8 and Figs. 9A to C. A significant increase was confirmed compared to the untreated plants. These results suggested that OS2C treatment may promote growth of poplar shoots and roots.

A full image of growth of the OS2C-treated poplar approximately 4 months after potting was compared with the untreated poplar, as shown in Fig. 10. With the untreated plants at approximately 4 months after potting, a transition to the dormant state was observed, including winter bud formation, growth arrest and leaf yellowing, whereas no such phenomenon was seen with the OS2C-treated plants at approximately 4 months after potting, and the leaves were green and had satisfactory continuous elongation growth. These results suggested that symbiosis with OS2C produces an inhibiting effect against dormancy and a preventative effect against growth arrest.

### [Example 5: Treatment of eucalyptus with actinomycetes strain OS2C]

The eucalyptus seeds (Eucalyptus Globulus) used were sterile seeds obtained by sterilization for 30 minutes with Kitchen Haiter diluted to 1/5, for 30 seconds with 70% ethanol, and for 15 minutes with Kitchen Haiter diluted to 1/5, followed by washing treatment. After seeding in 1% agar medium containing 1% sucrose and 1/4 MS medium and dark culturing for 2 days at 22°C with the plate upright, it was light cultured for 8 days under conditions with a light cycle of 16 hours light period/8 hours dark period, after which it was transplanted into soil that had been pre-sterilized with an autoclave.

During the same day, the OS2C spore suspension at a cell concentration of 10⁸ CFU/mL prepared in Example 1 was added at 1 mL per eucalyptus plant, close to the base of the transplant location. After 43 days, strain OS2C at the same cell concentration was further used for treatment at 1 mL per eucalyptus plant.

A full image of the eucalyptus plant was taken at 142 days after initial treatment with strain OS2C (152 days after seeding), and the root dry weight per plant was compared with the untreated plant, as shown in Fig. 11A and Fig. 11B. The dry weight of the roots increased by about 54% compared to the untreated group.

Four days after soil transplanting of a eucalyptus plant that had already been transplanted into a pot of larger size than in the treatment test described in the previous two paragraphs, the OS2C spore suspension at a cell concentration of 10⁸ CFU/mL prepared in Example 1 was added at 1 mL each per eucalyptus plant, close to the base of the transplant location. After 32 days, treatment was carried out again using the same OS2C strain.

A eucalyptus cultivation pot at 91 days after initial OS2C treatment (105 days after seeding) was prepared, and the number of leaves and dry weights per plant were compared with an untreated plant, as shown in Figs. 12 and Figs. 13A to C. The number of leaves, the shoot dry weight and the root dry weight were increased by about 56%, 12% and 58%, respectively. These results suggested that symbiosis of strain OS2C contributes to eucalyptus growth, and particularly to increased biomass production of the aboveground part via a root growth-promoting effect.

A eucalyptus cultivation pot at 159 days after initial OS2C treatment (180 days after seeding) was prepared, and dry weights of the aboveground part, stem alone and belowground part per plant were compared with untreated plants, as shown in Fig. 14 and Figs. 15A to C. The dry weight of the total aboveground part, the dry weight of only stem alone without leaves, and the dry weight of the belowground part increased by about 37%, 51% and 2-fold, respectively. The thickness of the trunk at a location 2 cm from the ground surface was compared between the OS2C-treated plants and untreated plants, as shown in Fig. 15D. For trunk diameter as well, the OS2C-treated plants tended to be larger than the untreated plants. These results were the same as the results shown in Fig. 12, suggesting that symbiosis with strain OS2C has an effect of increasing wood mass during prolonged growth of eucalyptus.

### [Example 6: Field test for rice seedlings treated with actinomycetes strain OS2C]

The rice seeds used (variety: Akita Komachi and Hokuriku 193) were sterile seeds obtained by sterilization for 1 minute with 70% ethanol and 30 minutes with 1% sodium hypochlorite, followed by washing treatment, after selection of paddy seeds by saltwater separation. The seeds were immersed in water to induce germination for 3 days, and the germinated seeds were seeded on a cell tray containing synthetic soil (trade name: Inaho Granular Soil), at one seed per cell.

The series of cultivation steps was carried out in a growth chamber (28°C, light cycle: 16 hours light period/8 hours dark period; approximately 22,000 lux). At 7 days after germination, the OS2C spore suspension at a cell concentration of 10⁹ CFU/mL prepared in Example 1 was applied to the seed surface at 1 mL per rice seed.

The Akita Komachi and Hokuriku 193 rice seedlings 19 days after OS2C treatment (26 days after germination), at the time of paddy field transplant in the field test of Example 6, were compared with untreated rice seedlings, as shown in Fig. 16A and Fig. 16B. The results demonstrated that OS2C treatment promoted early growth of seedlings. The early growth promoting effect was particularly notable with Akita Komachi.

Paddy transplantation was in early June in Akita Prefecture, and outdoor cultivation was continued until early October 10th (harvest stage). The amount of fertilizer applied to the paddy field before seedling transplantation, and herbicide dispersion before and after seedling transplantation, were carried out under common cultivation conditions.

At 78 days after OS2C treatment (59 days after paddy transplantation; vegetative growth stage), photographs were taken of Akita Komachi and Hokuriku 193 from above the paddy, and compared with untreated rice during the same period, as shown in Fig. 17A and Fig. 17B. Also, the average number of tillers of the OS2C-treated Akita Komachi and Hokuriku 193 rice at 78 days after OS2C treatment (59 days after paddy transplantation) were compared with the untreated rice, as shown in Fig. 17C and Fig. 17D. The average values for a total of 16 plants in each of the four groups are shown. In the OS2C-treated group, the average number of tillers increased by about 20% compared to the untreated group. These results clearly showed a significant increase in the average number of tillers in the aboveground part in both varieties, even after cultivation for about 2 months in a paddy field with OS2C treatment of the seedlings.

Akita Komachi and Hokuriku 193 (4 to 6 plants) at 155 days after OS2C treatment (136 days after paddy transplant; harvest stage) were compared with the untreated rice during the same period, as shown in Fig. 18A and Fig. 18B. The results demonstrated that in the rice harvest stage as well, OS2C treatment promoted rice growth in both varieties.

The fresh weight (FW) of the shoots, as a parameter of plant biomass volume, and the number and fresh weight (FW) of the panicles, as parameters of yield, after 155 days of OS2C treatment (136 days after paddy transplant; harvest stage) were compared with the untreated rice, as shown in Figs. 19A to C. Shown are the averages for a total of 31 to 40 plants (N = 31 to 40) in the four Akita Komachi groups, and the averages for a total of 74 plants (N = 74) in the four Hokuriku 193 groups. The results demonstrated that plant biomass volume of the shoots increased and rice harvest yield significantly increased by OS2C treatment of the seedlings, for both varieties.

### [Example 7: Comparative test of root hairs and lateral roots of rice treated with actinomycetes strain OS2C]

The rice seeds used (variety: Nipponbare) were sterile seeds obtained by sterilization for 1 minute with 70% ethanol and 30 minutes with 1% sodium hypochlorite, followed by washing treatment, after dehusking of paddy seeds which had been screened by saltwater separation. At 3 days after seeding in 0.5% agar medium (no components other than agar), the seedlings were transplanted into a sterilized No. 2 square dish holding 1/2 MS solid medium containing 0.5% gellan gum, at four per dish.

On the same day, before transplanting, they were coated with the OS2C spore suspension having a cell concentration of 10⁹ CFU/mL prepared in Example 1, at 150 µL per dish. The series of cultivation steps was carried out in a growth chamber (28°C, light cycle: 16 hours light period/8 hours dark period; approximately 22,000 lux).

The root hairs of the first seed roots at a distance of 4 cm from the seeds 3 days after OS2C treatment (6 days after seeding) were compared with the untreated rice, as shown in Fig. 20. In the OS2C-treated group, the root hairs were clearly longer than the untreated plants, demonstrating that differentiation and development of root hairs were accelerated within a short period after treatment.

Full images of root development of the rice at 7 days after OS2C treatment (10 days after seeding) were compared with the untreated rice, as shown in Fig. 21A. The total number of lateral roots and the lengths of the lateral roots per plant at 7 days after OS2C treatment (10 days after seeding) were compared with the untreated rice, as shown in Fig. 21B and Fig. 21B. The average values for the 8 plants are shown. In the OS2C-treated group, the lateral root lengths of the rice increased by about 14% compared to the untreated group. The results demonstrated that the root lengths were significantly longer in the OS2C-treated group compared to the untreated plants, with accelerated elongation growth as well.

### INDUSTRIAL APPLICABILITY

The present invention has very high utility, especially in the field of cultivating crop plants such as agricultural crops and resource crops, and economically important plants such as houseplants.

Strain OS2C has been deposited at the independent administrative National Institute of Technology and Evaluation, NITE Patent Microorganisms Depositary (Address: Room 122, 2-5-8 Kazusa Kamatari, Kisarazu City, Chiba Prefecture, Japan, 292-0818), as Accession No. NITE P-03739 (Original deposit date: September 6, 2022). Request was later made for transfer of the microorganism to an international depository based on the Budapest Treaty, dated November 11, 2024 (Receipt No.: NITE ABP-03739).

## Claims

1. A method for cultivating a plant, the method comprising applying an actinomycete belonging to *Streptomyces thermocarboxydus* to the plant or its propagating material.

2. The method according to claim 1, wherein the plant is an annual plant, and the actinomycete is applied to the plant from the 6th week after germination.

3. The method according to claim 1, wherein the plant is a perennial plant.

4. The method according to any one of claims 1 to 3, wherein the plant propagating material is part or all of the plant.

5. The method according to any one of claims 1 to 4, wherein the part of the plant is selected from among cells, tissue, organs, seeds, bulbs, cuttings and seedlings.

6. The method according to any one of claims 1 to 5, wherein the actinomycete is strain OS2C (Receipt No.: NITE ABP-03739) or a variant thereof.

7. The method according to any one of claims 1 to 6, wherein application of the actinomycete is accomplished by direct application of the actinomycete to the plant or its propagating material, or by indirect application of the actinomycete to the surrounding environment of the plant or its propagating material.

8. The method according to any one of claims 1 to 7, wherein application of the actinomycete improves the growth condition of the plant.

9. The method according to claim 8, wherein the improvement in the growth condition of the plant is increased crop harvest yield, increased plant biomass volume, promoted root formation, promoted development of roots, stems or leaves, suppressed dormancy of the plant, suppressed growth arrest of the plant, or any combination of the foregoing.

10. A plant or its propagating material, created by a method according to any one of claims 1 to 9.

11. A composition comprising an actinomycete belonging to *Streptomyces thermocarboxydus,* which is used for improving the growth condition of a plant.

12. The composition according to claim 11, wherein the actinomycete is strain OS2C (Receipt No.: NITE ABP-03739) or a variant thereof.

13. The composition according to claim 11 or 12, wherein the improvement in the growth condition of the plant is increased crop harvest yield, increased plant biomass volume, promoted root formation, promoted development of roots, stems or leaves, suppressed dormancy of the plant, suppressed growth arrest of the plant, or any combination of the foregoing.

14. The composition according to any one of claims 11 to 13 above, wherein the plant is an annual plant, and the composition is applied to the plant from the 6th week after germination.

15. The composition according to any one of claims 11 to 13, wherein the plant is a perennial plant.
